# EUROPEAN PATENT SPECIFICATION

(11) **EP 4 585 195 B1**
(45) Date of publication and mention of the grant of the patent: **07.10.2026**
(21) Application number: 24219385.2
(22) Date of filing: 12.12.2024
(51) Int. Cl.: A61F 13/00, A61F 13/02

(54) **SHIELDING DEVICE**
ABSCHIRMVORRICHTUNG
DISPOSITIF DE BLINDAGE

(43) Date of publication of application: 16.07.2025
(73) Proprietor: Prosys International Ltd, London SW19 5 AP (GB)
(72) Inventor: STEER, Graham E., London, SW 19 5 AP (GB)
(74) Representative: dompatent

(56) References cited:
- US-A1- 2013 231 621
- US-B1- 6 334 443
- US-B2- 7 622 629

## Description

The present invention is related to a shielding device for protecting an application area of a patient's skin. The intended application area where the shielding device of the current invention can be applied to can be generally a wound site of a patient, all infusion sites for parenteral feeding, oncology fluid infusion sites, all chemotherapy, immunology and hormone therapy PIC and shunt line sites, all vascular access sites for dialysis, all ports under skin, also all areas where the shielding device covers body worn pumps and meter and injectors.

In the field of medical procedures, it is common to use shielding devices such as plasters and/or wound covers to protect the patient's skin and wounds from exposure to the external surrounding and help to prevent that external fluids or particles reach the wound area and maintain a sterile environment. Traditional shielding devices often consist of multiple layers that are joined together to form an inseparable layer stack including a wound dressing on one surface of the shielding device which is adhered to the skin around the application area. These devices typically include an adhesive layer to secure the device to the skin and a protective layer to cover the application area. One major drawback is that the known shielding devices are flat and lack the ability to form an enclosure with a dedicated height above the application area. This limitation makes them unsuitable for shielding ports or other medical devices attached to the patient which require a protective enclosure that extends above the skin surface. As a result, these flat shielding devices fail to provide adequate protection and can lead to contamination of the application area. US 7 622 629 B2 provides a wound shield for protecting wounds comprising a frame circumscribing wound in spiral forming at least two wraps; cover engaging the frame forming enclosed wound space within the frame spiral, where the wraps have different heights.

Taken aforementioned disadvantages of the known prior art, it is the object of the current invention to overcome the disadvantages of the prior art by providing a shielding device that ensures a fluid-tight seal around the application area, minimizes skin irritation, and is easy to apply and remove. Importantly, the invention addresses the need for a shielding device that can form an enclosure with a dedicated height to protect ports or other medical devices attached to the patient.

The object of the current invention is solved by a shielding device for protecting an application area of a patient's skin. The shielding device comprises a first upper layer extending along an extension plane being delimited by an outer contour and a second collar layer extending along an extension plane and formed in a ring shape along the extension plane having an outer size and outer contour identical to the outer contour of the first upper layer and having a ring width along the extension plane forming an inner ring contour. The first upper layer and the second collar layer are laid on top of each other with matching outer contours and are joined together in the area of their outer contours to form a fluid tight pouch between the first upper layer and the second collar layer, wherein the pouch has a lower opening formed by the inner ring contour of the second collar, wherein the second collar layer comprises an adhesive seal on a second surface of the extension plane facing away from the pouch and wherein the adhesive seal extends circumferentially around the inner ring contour and is adapted to removably adhere the shielding device to the patient's skin to surround an application area of the patient and to form a fluid tight enclosure above the application area.

The extension plane of the first upper layer has a first surface facing away from the pouch and a second opposite surface facing toward the pouch and forming a first partial area of the inner wall of the pouch. The extension plane of the second collar layer has a first surface facing toward the pouch and forming a second partial area of the inner wall of the pouch and a second surface facing away from the pouch. The first upper layer and the second collar layer are each formed by a thin plastic film material wherein the plastic film material is fluid tight. Preferably, the plastic film material of the first upper layer and/or the second collar layer are transparent.

The fluid tight pouch is formed between the second surface of the first upper layer and the first surface of the second collar layer. The pouch being delimited to the outer side by the joint between the first upper layer and the second collar layer in the area of/around their outer contours. The first upper layer and the second collar layer are only joined in the area of the outer contour, otherwise, the two layers are not connected to each other.

When the claimed shielding device is applied to the patient's skin in an application area, the fluid tight enclosure is formed between the skin of the patient and the pouch, wherein the opening of the pouch is sealed to the patient's skin in a fluid tight manner by application of the adhesive seal to the patient's skin to surround the application area/application site. The adhesive seal enables to form with the skin of the patient at the application site an enclosed protective room formed by the pouch. The pouch which is itself formed by and between the first upper shield layer and the second collar layer is able to adapt its inner pouch volume and size during the application of the device to the wound site by unfolding/distancing the first upper layer with respect to the second collar layer.

This shielding device ensures a fluid-tight seal to maintain a sterile environment for the application site and features a secure, removable adhesive for reliable attachment. Its ring-shaped collar allows for versatile application across different application areas and application scenarios, enhancing its adaptability. Overall, it combines robust protection with patient comfort and practical usability.

The outer contour and the size of the first upper and the second collar layer are adapted to the specific application site of a patient. The adaption to the application area can be achieved, for example, by an adaption of the size, the geometry, the elasticity of the upper layer and the second collar layer. The outer contours can be formed as square or rectangular with rounded edges, alternatively a round or elliptical outer contour can be foreseen for the layers.

The inner counter of the second collar layer in the extension plane can be provided to extend in parallel to the outer contour of the second collar layer, whereby the second collar layer has a consistent ring width. Nevertheless, according to the current invention, it can be also provided that the second collar layer has a varying ring width along the contour so that the inner ring contour does not extend in parallel to the outer ring contour.

The adhesive seal preferably does not cover the whole surface of the second surface but only covers a ring-shaped partial area of the second surface, most preferably neighboured to the ring contour of the collar layer. The adhesive seal circumferentially surrounds the inner ring contour and thereby, the lower opening of the pouch of the shielding device.

The adhesive seal can be formed by a third foam layer formed in a ring shape with a first surface and a second surface, wherein the first surface of the third foam layer is joined together with the second surface of the second collar layer along a ring shaped partial area of the first surface and wherein the second surface of the foam layer is coated with an adhesive at least in a partial area of the second surface which is adapted to removably adhere the shielding device to the patient's skin and to form a fluid tight seal with the patient's skin. The third foam layer can be preferably formed by a flexible and deformable foam material, most preferably by a shape memory polymer. The third foam layer enhances patient comfort and protects the skin from irritation, while its adhesive coating ensures a secure, fluid-tight seal of the shielding device to the application area.

The third foam layer is formed by a foamed plastic material, wherein the foamed plastic material is fluid tight. Preferably, the foam material is formed with closed surfaces. Most preferably, the third foam layer can be formed by a closed cell foam layer made from Ethylene Vinyl Acetate (EVA). Preferably, the third foam layer is identical in size and shape to the second collar layer. The joint between the third foam layer and the second collar layer is fluid tight.

The adhesive of the adhesive seal can be formed by a solvent based water stable acrylic adhesive. The adhesive according to the invention is adapted to be applied to the skin of a patient over an extended period of time and is adapted to ensure a water-tight seal of the shielding device to the skin, whereby the adhesive is water stable.

The third foam layer can be formed by an Ethylene Vinyl Acetate, EVA, foam material, preferably with a thickness between the first and the second surface of 500 micron.

It can be foreseen that the first upper layer and/or the second collar layer is/are formed by a clear Ethylene Vinyl Acetate, EVA, film, preferably with a layer thickness of 75 micron. The EVA film has the advantage of being highly flexible, moisture-resistant, and durable, making it ideal for wound coverings that need to conform to the body and protect wounds from external contaminants. Additionally, its softness and strong adhesion ensures comfort and secure placement, while providing UV protection for exposed wounds.

The layer thickness of each layer is measured as the shortest distance between the respective first and second surface of the specific layer.

Preferably, the adhesive seal is covered by a fourth release liner layer.

The fourth release liner layer can be formed by a siliconized releasable paper liner. The siliconized releasable paper liner protects the adhesive of the device from contamination and ensures easy, clean application. This maintains the adhesive's performance and compliance with regulatory standards, enhancing the device's reliability and effectiveness. The smooth, non-stick surface of a siliconized liner ensures easy and clean application by allowing the liner to be removed without pulling or damaging the adhesive.

The fourth release liner layer has an outer contour extending beyond the outer contour of the second collar layer. The fourth release liner extending beyond the outer contour of the second collar layer enables to form a grasping area of the release liner to easily peel off the release liner from the adhesive seal to uncover the adhesive seal prior to its application to the patient's skin.

More preferably, the fourth release liner comprises an additional tab which protrudes from an extension plane of the fourth release paper liner to the underside of the shielding device and provides a grip area to grasp and peel the fourth release liner from the adhesive.

Preferably, the first upper layer and the second collar layer are joined together via a high frequency welding to form a weld along the outer contour. The weld forms the fluid tight joint between the first upper layer and the second collar layer and thereby creates the fluid tight pouch. High frequency welding creates a strong, durable, and fluid-tight seal between the first upper layer and the second collar layer, ensuring reliable protection. This method also provides precision and efficiency in manufacturing, enhancing the device's overall quality and consistency.

The third foam layer can be preferably joined together with the second collar layer along a ring-shaped partial area via high frequency welding. In this preferred embodiment, the weld between the third foam layer and the second collar layer again forms a fluid tight joint. As the joint circumferentially surrounds the inner ring of the second collar layer, the third foam layer is joined in a fluid tight manner to the pouch.

According to a preferred embodiment, it can be foreseen that the first upper layer comprises at least one marking to label a snip of area in the area of the outer contour for creating a draining opening. Preferably, the at least one mark shows a cutting line with a scissor in order to mark an area which should be cut off by a scissor to create a drain opening to the pouch of the shielding device. The marking on the first upper layer guides users on where to cut to create a drainage opening.

A second aspect of the current invention is directed to a wound drainage kit comprising a shielding device with the features according to the first aspect of the invention and further comprises a closure device. The closure device is formed by an elastic strip of a foam material layer which is supported by a plastically deformable support wire covered or enclosed in the foam material and wherein a first side of the elastic strip is formed with an adhesive layer or coating.

A third aspect which does not form part of the current invention concerns the usage of a shielding device with the features according to the first aspect for protecting a wound site and/or an exposed tissue of a patient from exposure to external influences such as to protect the wound site from bacterial ingress and/or from ingress of fluids or particles. Thereby, the shielding device allows a patient of the device to bath and/or shower wherein the wound site in the area of the application area being protected from ingress such as of pathogens.

According to a further aspect which does not form part of the current invention concerns the usage of a shielding device according to the first aspect for covering a catheter fitted into a patient's skin to protect the catheter site from exposure to external influences such as fluid and/or bacterial ingress.

Yet, another aspect which does not form part of the current invention concerns a usage of the shielding device of the first aspect for covering a feeding system fitted onto a patient's skin to protect the site of the feeding system from exposure to external influences such as fluid and/or bacterial ingress.

A further preferred aspect which does not form part of the current invention concerns the usage of a shielding device with the features of the first aspect of the invention for collecting and draining wound fluid by adhering the shielding device to the patient's skin to surround a wound of the patient as application area for a defined application time to collect fluid eventually released from the wound in the pouch and draining the collected wound from the pouch by snipping off a partial area of the outer contours or puncturing the first upper layer or the second collar layer to form a drainage opening.

A further aspect which does not form part of the current invention concerns the usage of a wound drainage kit with the features according to the second aspect of the current invention for collecting and draining wound fluid by adhering the shielding device to the patient's skin to surround a wound of the patient as an application area for a defined application time to collect fluid eventually released from the wound in the pouch and draining the collected wound fluid from the pouch by snipping off a partial area of the outer contours or puncturing the first upper layer or the second collar layer to form a drainage opening and adhering a closure device to the first upper layer and/or the second collar layer of the shielding device in the area of the drainage opening and sealing the drainage opening by folding the first upper layer and/or the second collar layer around the closure device and plastically deforming the shielding device to close the drainage opening.

In the following, reference will be made to the attached figures describing exemplary embodiments of the shielding device according to the present invention.
- Fig. 1: shows a perspective view of a first exemplary embodiment of a shielding device according to the current invention;
- Fig. 2: shows a bottom view on the underside of the exemplary shielding device according to Fig. 1;
- Fig. 3: shows a cut view of the exemplary embodiment of the shielding device according to Fig. 1 including an enlarged partial view.

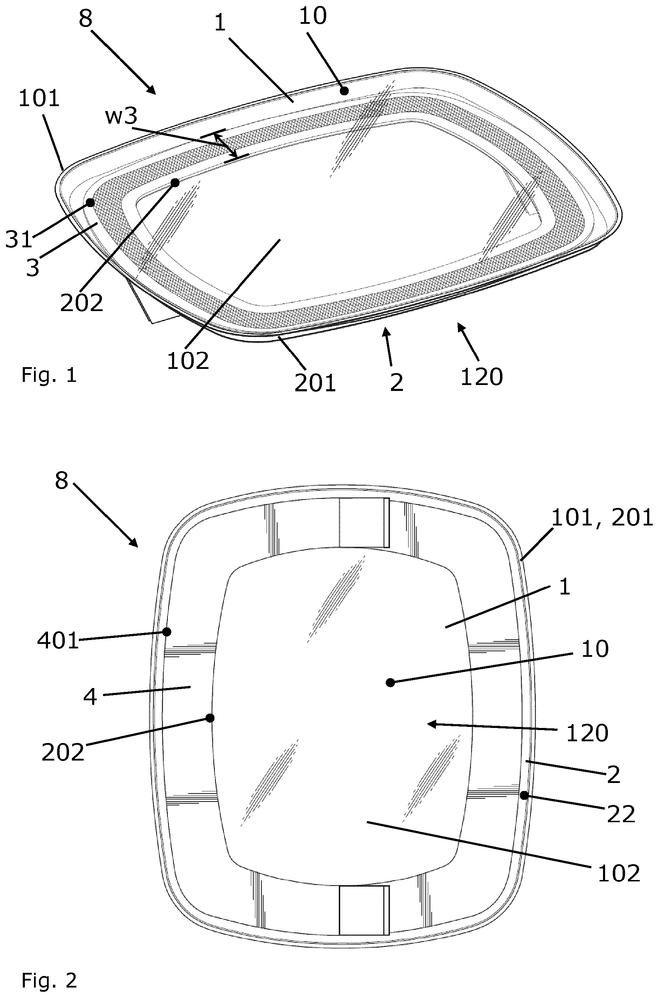

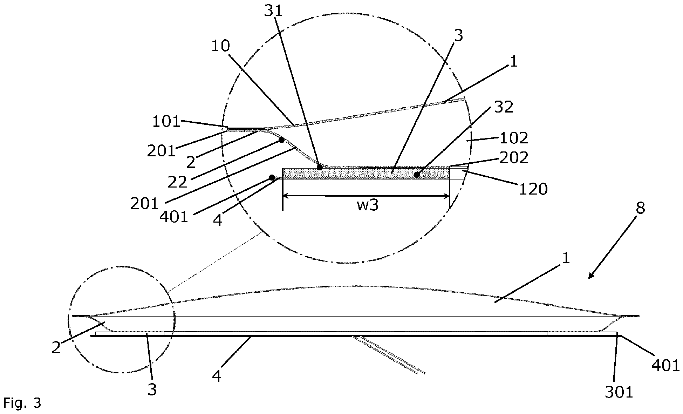

As can be taken from the Figures 1-3, the shielding device 8 adapted to protect an application area of a patient's skin comprises a first upper layer 1 extending along an extension plane 10 and being delimited by an outer contour 101 and a second collar layer 2 extending along an extension plane 20 and formed in a ring shape having an outer size and outer contour 201 identical to the outer contour 101 of the first upper layer 1. The second collar layer 2 has a ring width along the extension plane 20 and forming an inner ring contour 202. The first upper layer 1 and the second collar layer 2 are laid on top of each other with matching outer contours 101, 201 wherein the first upper layer 1 and the second collar layer 2 are joined together in the area of the outer contours 101, 201 of the layers 1 and 2 to form a fluid tight pouch 102 between the first upper layer 1 and the second collar layer 2.

In the exemplary embodiment as shown in the figures, the first upper layer 1 as well as the second collar layer 2 are each formed by a transparent film layer, the layers 1, 2 can be, for example, formed by a clear/transparent Ethylene Vinyl Acetate, EVA film.

The second collar layer 2 comprises an adhesive seal 3 on a second surface 22 of the extension plane 20 facing away from the pouch 102 and wherein the adhesive seal 3 extends circumferentially around the inner ring contour 202 and is adapted to removably adhere the shielding device 8 to the patient's skin to surround an application area of the patient and to form a fluid tight enclosure above the application area.

In the exemplary embodiment as shown in the figures, the adhesive seal 3 is formed by a third foam layer which is formed in a ring shape with a first surface 31 and a second surface 32 wherein the first surface 31 of the third foam layer 3 is joined together with a second surface 22 of the second collar layer 2 along a ring-shaped partial area of the first surface 31 and wherein the second surface 32 of the foam layer 3 is coated with an adhesive at least in a partial area of the second surface 32 and adapted to removably adhere the shielding device 8 to the patient's skin and to form a fluid-tight seal with the patient' skin. The third foam layer can be formed in identical size and shape to the second collar layer 2. The exemplary embodiments according to the Figures 1-4 show an embodiment wherein the third foam layer 3 is provided with a smaller outer diameter and a smaller outer contour, thereby being recessed in the extension plane from the outer contour of the second collar layer 2. The third foam layer has a ring width w3 along its extension plane. The third foam layer 3 can be formed by an elastically foamed, preferably closed, cell material and can also be manufactured by an Ethylene Vinyl Acetate, EVA foam.

The exemplary embodiment shows the optional feature that the adhesive seal 3 is covered by a fourth release liner layer 4 wherein the aforementioned release liner layer 4 can be, for example, formed by a siliconized release paper liner.

## Claims

1. Shielding device (8) for protecting an application area of a patient's skin comprising:
a first upper layer (1) extending along an extension plane (10) being delimited by an outer contour (101), and
a second collar layer (2) extending along an extension plane (20) formed in a ring shape having an outer size and an outer contour (201) identical to the outer contour (101) of first upper layer (1) and having a ring width (w2) along the extension plane (20) forming an inner ring contour (202),
wherein the first upper layer (1) and the second collar layer (2) are laid on top of each other with matching outer contours (101, 201) and joined together in the area of their outer contours (101, 201) to form a fluid tight pouch (102) between the first upper layer (1) and the second collar layer (2);
wherein the pouch (102) has a lower opening (120) formed by the inner ring contour (202) of the second collar layer (2);
wherein the second collar layer (2) comprises an adhesive seal (3) on a second surface (22) of the extension plane (20) facing away from the pouch (102); and
wherein the adhesive seal (3) extends circumferential around the inner ring contour (202) and is adapted to removably adhere the shielding device (8) to the patient's skin to surround an application area of the patient and to form a fluid tight enclosure above the application area.

2. Shielding device (8) according to claim 1, wherein the adhesive seal (3) is formed by a third foam layer (3) formed in a ring shape with a first surface (31) and a second surface (32), wherein the first surface (31) of the third foam layer (3) is joined together with the second surface (22) of the second collar layer (2) along a ring shaped partial area of the first surface (31) and wherein the second surface (32) of the foam layer (3) is coated with an adhesive at least in a partial area of the second surface (32) adapted to removably adhere the shielding device (8) to the patient's skin and to form a fluid tight seal with the patient's skin.

3. Shielding device (8) according to claim 1 or 2, wherein the third foam layer (3) is identical in size and shape to the second collar layer (2).

4. Shielding device (8) according to any one of the preceding claims, wherein the adhesive of the adhesive seal (3) is formed by a solvent based water stable acrylic adhesive.

5. Shielding device (8) according to anyone of claims 2 to 4, wherein the third foam layer (3) is formed by an Ethylene Vinyl Acetate, EVA, foam material, preferably with a thickness (t3) between the first and the second surface (31, 32) of 500 micron.

6. Shielding device (8) according to any one of the preceding claims, wherein the first upper layer (1) and/or the second collar layer (2) is/are formed by a clear Ethylene Vinyl Acetate, EVA, film, preferably with a layer thickness of 75 micron.

7. Shielding device (8) according to any one of the preceding claims, wherein the adhesive seal (3) is covered by a fourth release liner layer (4).

8. Shielding device (8) according to claim 7, wherein the fourth release liner layer (4) is formed by a siliconized releasable paper line (4).

9. Shielding device (8) according to claim 7 or 8, wherein the fourth release liner layer (4) has an outer contour (401) extending beyond the outer contour (301) of the third foam layer (3).

10. Shielding device (8) according to any one of the preceding claims, wherein the first upper layer (1) and the second collar layer (2) are joined together via high frequency welding to form a weld along the outer contour (101, 201).

11. Shielding device (8) according to any one of the claims 2 to 10, wherein the third foam layer (2) is joined together with the second collar layer (2) along a ring-shaped partial area via high frequency welding.

12. Shielding device (8) according to any one of the claims 1 to 11, wherein the first upper layer (1) comprises at least one marking to label a snip off area in the area of the outer contour (101) for creating a draining opening (1020).

## Patentansprüche

1. Abschirmvorrichtung (8) zum Schutz eines Auftragungsbereichs der Haut eines Patienten, aufweisend:
eine erste obere Schicht (1), die sich entlang einer Ausdehnungsebene (10) erstreckt, die durch eine Außenkontur (101) begrenzt wird, und
eine zweite Kragenschicht (2), die sich entlang einer Ausdehnungsebene (20) erstreckt, die in einer Ringform ausgebildet ist und eine Außenabmessung sowie eine Außenkontur (201) aufweist, die mit der Außenkontur (101) der ersten oberen Schicht (1) identisch ist, und die entlang der Ausdehnungsebene (20) eine Ringbreite (w2) aufweist, die eine innere Ringkontur (202) bildet,
wobei die erste obere Schicht (1) und die zweite Kragenschicht (2) mit übereinstimmenden Außenkonturen (101, 201) übereinandergelegt und im Bereich ihrer Außenkonturen (101, 201) zusammengefügt sind, um einen fluiddichten Beutel (102) zwischen der ersten oberen Schicht (1) und der zweiten Kragenschicht (2) zu bilden;
wobei der Beutel (102) eine untere Öffnung (120) aufweist, die durch die innere Ringkontur (202) der zweiten Kragenschicht (2) gebildet wird;
wobei die zweite Kragenschicht (2) eine Klebedichtung (3) auf einer zweiten Oberfläche (22) der Ausdehnungsebene (20) aufweist, die vom Beutel (102) abgewandt ist; und
wobei sich die Klebedichtung (3) in Umfangsrichtung um die innere Ringkontur (202) erstreckt und dazu ausgelegt ist, die Abschirmvorrichtung (8) lösbar an der Haut des Patienten festzukleben, um einen Auftragungsbereich des Patienten zu umschließen und eine fluiddichte Umhüllung über dem Auftragungsbereich zu bilden.

2. Abschirmvorrichtung (8) nach Anspruch 1, wobei die Klebedichtung (3) durch eine dritte Schaumschicht (3), die ringförmig ausgebildet ist, mit einer ersten Oberfläche (31) und einer zweiten Oberfläche (32) ausgebildet ist, wobei die erste Oberfläche (31) der dritten Schaumschicht (3) entlang eines ringförmigen Teilbereichs der ersten Oberfläche (31) mit der zweiten Oberfläche (22) der zweiten Kragenschicht (2) zusammengefügt ist, und wobei die zweite Oberfläche (32) der Schaumschicht (3) zumindest in einem Teilbereich der zweiten Oberfläche (32) mit einem Klebstoff beschichtet ist, der dazu ausgelegt ist, die Abschirmvorrichtung (8) lösbar auf der Haut des Patienten festzukleben und eine fluiddichte Abdichtung mit der Haut des Patienten zu bilden.

3. Abschirmvorrichtung (8) nach Anspruch 1 oder 2, wobei die dritte Schaumschicht (3) in Größe und Form mit der zweiten Kragenschicht (2) identisch ist.

4. Abschirmvorrichtung (8) nach einem der vorstehenden Ansprüche, wobei der Klebstoff der Klebedichtung (3) aus einem lösungsmittelbasierten, wasserbeständigen Acrylklebstoff besteht.

5. Abschirmvorrichtung (8) nach einem der Ansprüche 2 bis 4, wobei die dritte Schaumschicht (3) aus Ethylen-Vinylacetat-, EVA, Schaumstoff besteht, vorzugsweise mit einer Dicke (t3) zwischen der ersten und der zweiten Oberfläche (31, 32) von 500 Mikrometer.

6. Abschirmvorrichtung (8) nach einem der vorstehenden Ansprüche, wobei die erste obere Schicht (1) und/oder die zweite Kragenschicht (2) aus einer transparenten Ethylen-Vinylacetat-, EVA, Folie besteht bzw. bestehen, vorzugsweise mit einer Schichtdicke von 75 Mikrometer.

7. Abschirmvorrichtung (8) nach einem der vorstehenden Ansprüche, wobei die Klebedichtung (3) von einer vierten Trennfolienschicht (4) bedeckt ist.

8. Abschirmvorrichtung (8) nach Anspruch 7, wobei die vierte Trennfolienschicht (4) aus einem silikonisierten ablösbaren Trennpapier (4) besteht.

9. Abschirmvorrichtung (8) nach Anspruch 7 oder 8, wobei die vierte Trennfolienschicht (4) eine Außenkontur (401) aufweist, die über die Außenkontur (301) der dritten Schaumschicht (3) hinausragt.

10. Abschirmvorrichtung (8) nach einem der vorstehenden Ansprüche, wobei die erste obere Schicht (1) und die zweite Kragenschicht (2) durch Hochfrequenzschweißen zusammengefügt sind, um eine Schweißnaht entlang der Außenkontur (101, 201) zu bilden.

11. Abschirmvorrichtung (8) nach einem der Ansprüche 2 bis 10, wobei die dritte Schaumschicht (2) entlang eines ringförmigen Teilbereichs durch Hochfrequenzschweißen mit der zweiten Kragenschicht (2) zusammengefügt ist.

12. Abschirmvorrichtung (8) nach einem der Ansprüche 1 bis 11, wobei die erste obere Schicht (1) mindestens eine Markierung aufweist, um einen Abschneidebereich im Bereich der Außenkontur (101) zur Herstellung einer Ablauföffnung (1020) zu kennzeichnen.

## Revendications

1. Dispositif de protection (8) destiné à protéger une zone d'application de la peau d'un patient, comprenant :
une première couche supérieure (1) s'étendant le long d'un plan d'étendue (10) qui est délimitée par un contour extérieur (101), et
une deuxième couche de collerette (2) s'étendant le long d'un plan d'étendue (20) formée en une forme d'anneau ayant une taille extérieure et un contour extérieur (201) identique au contour extérieur (101) de la première couche supérieure (1) et ayant une largeur d'anneau (w2) le long du plan d'étendue (20) formant un contour intérieur d'anneau (202),
dans lequel la première couche supérieure (1) et la deuxième couche de collerette (2) sont posées l'une sur l'autre avec des contours extérieurs correspondants (101, 201) et sont liées l'une à l'autre dans la zone de leurs contours extérieurs (101, 201) pour former une poche étanche au fluide (102) entre la première couche supérieure (1) et la deuxième couche de collerette (2) ;
dans lequel la poche (102) comporte une ouverture inférieure (120) formée par le contour intérieur d'anneau (202) de la deuxième couche de collerette (2) ;
dans lequel la deuxième couche de collerette (2) comprend un matériau de scellement adhésif (3) sur une deuxième surface (22) du plan d'étendue (20) à l'opposé de la poche (102) ; et
dans lequel le matériau de scellement adhésif (3) s'étend de manière circonférentielle autour du contour intérieur d'anneau (202) et est conçu pour coller de manière amovible le dispositif de protection (8) à la peau d'un patient pour entourer une zone d'application du patient et pour former une enceinte étanche aux fluides sur la zone d'application.

2. Dispositif de protection (8) selon la revendication 1, dans lequel le matériau de scellement adhésif (3) est formé par une troisième couche de mousse (3) formée en une forme d'anneau avec une première surface (31) et une seconde surface (32), dans lequel la première surface (31) de la troisième couche de mousse (3) est liée à la seconde surface (22) de la deuxième couche de collerette (2) le long d'une zone partielle en forme d'anneau de la première surface (31) et dans lequel la seconde surface (32) de la couche de mousse (3) est revêtue d'un adhésif au moins en partie dans une zone partielle de la seconde surface (32) conçu pour coller de manière amovible le dispositif de protection (8) à la peau d'un patient et pour former une étanchéité aux fluides avec la peau du patient.

3. Dispositif de protection (8) selon la revendication 1 ou la revendication 2, dans lequel la troisième couche de mousse (3) est de taille et de forme identiques à celles de la deuxième couche de collerette (2).

4. Dispositif de protection (8) selon l'une quelconque des revendications précédentes, dans lequel l'adhésif du matériau de scellement adhésif (3) est formé par un adhésif acrylique stable à l'eau à base de solvant.

5. Dispositif de protection (8) selon l'une quelconque des revendications 2 à 4, dans lequel la troisième couche de mousse (3) est formée par un matériau de mousse à base de copolymère éthylène-vinyle acétate, dit EVA, ayant de préférence une épaisseur (t3) entre les première et seconde surfaces (31, 32) de 500 microns.

6. Dispositif de protection (8) selon l'une quelconque des revendications précédentes, dans lequel la première couche supérieure (1) et/ou la deuxième couche de collerette (2) est/sont formées par un film de copolymère éthylène-vinyle acétate, dit EVA, transparent ayant de préférence une épaisseur de couche de 75 microns.

7. Dispositif de protection (8) selon l'une quelconque des revendications précédentes, dans lequel le matériau de scellement adhésif (3) est recouvert d'une quatrième couche de couverture à décoller (4).

8. Dispositif de protection (8) selon la revendication 7, dans lequel la quatrième couche de couverture à décoller (4) est formée par une garniture en papier siliconé pouvant être décollée (4).

9. Dispositif de protection (8) selon la revendication 7 ou la revendication 8, dans lequel la quatrième couche de couverture à décoller (4) a un contour extérieur (401) s'étendant au-delà du contour extérieur (301) de la troisième couche de mousse (3).

10. Dispositif de protection (8) selon l'une quelconque des revendications précédentes, dans lequel la première couche supérieure (1) et la deuxième couche de collerette (2) sont liées l'une à l'autre par le biais d'un soudage haute fréquence pour former une soudure le long du contour extérieur (101, 201).

11. Dispositif de protection (8) selon l'une quelconque des revendications 2 à 10, dans lequel la troisième couche de mousse (2) est liée à la deuxième couche de collerette (2) le long d'une zone partielle en forme d'anneau par le biais d'un soudage haute fréquence.

12. Dispositif de protection (8) selon l'une quelconque des revendications 1 à 11, dans lequel la première couche supérieure (1) comprend au moins un marquage servant à repérer une zone à découper dans la zone du contour extérieur (101) de façon à créer une ouverture de drainage (1020).
